# EUROPEAN PATENT SPECIFICATION

(11) **EP 0 957 960 B1**
(45) Date of publication and mention of the grant of the patent: **01.09.2004**
(21) Application number: 96916747.7
(22) Date of filing: 03.06.1996
(51) Int. Cl.: A61M 15/00, A61M 11/06

(54) **BREATHING CIRCUIT APPARATUS FOR A NEBULIZER**
BEATMUNGSKREIS FÜR EINEN ZERSTÄUBER
APPAREIL A CIRCUIT RESPIRATOIRE POUR NEBULISEUR

(30) Priority: 07.06.1995 US 485880
(43) Date of publication of application: 24.11.1999
(73) Proprietor: SALTER LABS, Arvin, California 93203 (US)
(72) Inventor: SALTER, Peter, W., Tehachapi, CA 93561 (US); CHUA, James, Tehachapi, CA 93561 (US)
(74) Representative: Watts, Peter Graham
(86) International application number: PCT/US1996/007957
(87) International publication number: WO 1996/040333

(56) References cited:
- EP-A- 0 540 775
- EP-A- 0 626 180
- WO-A-86/01731
- US-A- 5 209 225
- US-A- 5 388 571

## Description

### FIELD OF THE INVENTION

The present invention relates to a breathing circuit apparatus adapted for use with a nebulizer device so that a user can inhale an aerosol generated by the nebulizer device from the breathing circuit apparatus. More particularly, the present invention is directed to a breathing circuit apparatus adapted for use with a nebulizer device that generates a medicament-containing aerosol so that a breathing user/patient can inhale the medicinal aerosol from the breathing circuit apparatus and subsequently exhale exhalation gas through the breathing circuit apparatus.

### BACKGROUND OF THE INVENTION

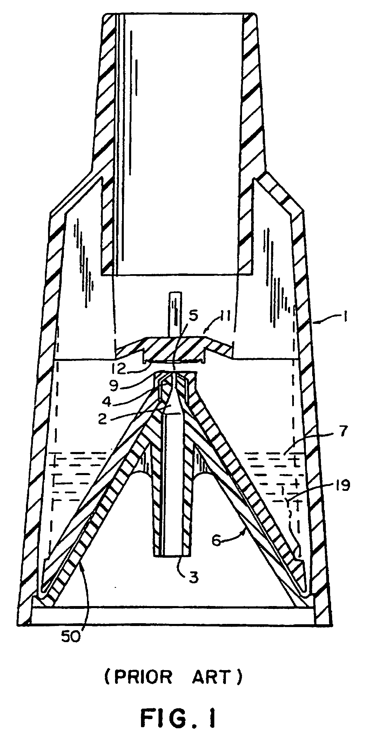

Various types of nebulizers have been used in commerce for a variety of applications and are well known in the art. One common application for a nebulizer is in the medical industry. Such a nebulizer has been used in the medical industry for effective delivery of medicines to a patient's lungs. An example of a nebulizer capable of delivering medicine to a patient's lungs is disclosed in U.S. Patent 4,746,067 to Steven A. Svoboda which is described generally as prior art with reference to Figure 1.

This prior art nebulizer is a device for aerosolizing a liquid medicine 7 with a pressurized gas (the source of which is not shown) which is typically compressed air. The prior art nebulizer includes a container 1 for holding liquid medicine 7, a mixing mechanism 50 and a deflector member 11. Mixing mechanism 50 comprises a venturi tube 2 and a liquid conduit means 6. Venturi tube 2 has an upstream opening 3 adapted for connection to the source of pressurized gas, a throat portion 4 and a discharge opening 5. Liquid conduit means 6 is connected to container 1 and has an outlet opening 8 adjacent to a downstream end 9 of throat portion 4. Venturi tube 2 is dimensioned and positioned to withdraw liquid medicine 7 from reservoir 19 and through liquid conduit means 6 and to project a mixture of liquid and gas out of discharge opening 5 at a high velocity in a nebulized liquid/gas stream.

A deflector member 11 is positioned adjacent to and disposed from discharge opening 5 of venturi tube 2 and has a surface 12 intersecting the liquid/gas stream and causing a dispersion of the liquid/gas stream in a second direction different from the first direction. Deflector member 11 is employed to further reduce the liquid droplet size in the nebulized liquid/gas stream thereby causing a fine mist which dispersed about an interior chamber of container 1.

With the prior art being replete with nebulizers, some other nebulizers are described in U.S. Patent 5,287,847 to Piper et al., U.S. Patent 5,209,225 to Glenn, U.S. Patent 4,657,007 to Calin et al., U.S. Patent 4,588,129 to Shanks, U.S. Patent 4,512, 341 to Lester, U.S. Patent 3,762,409 to Lester, U.S. Patent 3,744,722 to Burns and U.S. Patent 3,097,645. In one form or another, all of these patents as well as other prior art nebulizers teach a nebulizer device which mixes a pressurized aerosolizing gas with a liquid to produce a stream of nebulized liquid and gas predicated upon the commonly known venturi principle. Often, this stream is directed to a deflector member which further reduces the size of the liquid droplets in the nebulized liquid stream to form a fine mist. The mist disperses throughout the interior chamber of the nebulizer. Particularly in light of the patents noted above, the fine mist of nebulized liquid is inhaled by the user through a tube opened to the ambient air environment. Prior art nebulizers continuously produce the fine mist. Thus, during exhalation, some of the fine mist which typically contains costly medicament exits the container through the tube and into the ambient air environment. This results in wasted medicament.
There is a need in the industry for a breathing circuit apparatus which can be used in combination with a nebulizer device operative to generate an aerosol so that the user can inhale aerosol through his/her nose and/or mouth from the breathing circuit apparatus and thereafter can exhale exhalation gas through the breathing circuit apparatus and into ambient environment. It would be advantageous if the breathing circuit apparatus used in combination with a nebulizer device can create a preselected amount of positive back-pressure on the lungs when the user exhales through the breathing circuit apparatus. In this latter regard, it would also be advantageous if the breathing circuit apparatus included an adjustable outlet valve so that the amount of positive back-pressure on the lungs can be regulated, or be provided with a flapper valve with preselected stiffness for providing back-pressure of from about 5 cm to about 20 cm of water. There is also a need in the industry for breathing circuit apparatus used with a nebulizer device that minimizes loss of aerosolized medicament into the ambient environment to minimize waste of the medication contained in the aerosol and limit contamination of the atmosphere with medicament. It would be advantageous if the aerosolized medicament can be contained in the breathing circuit apparatus as much as possible, particularly during exhalation, so that a health care provider can better provide medicament dosage requirements for the user/patient. It would be further advantageous if the breathing circuit apparatus adapted for use with the nebulizer device has a mouthpiece structure.

These needs are addressed, to varying degrees, by EP-A-0540755; WO-A-8601731 and in particular EP-A-0626180 which forms the basis of the preamble of claim 1.

With a breathing circuit apparatus of this type adapted for both inhalation and exhalation by the patient, it is desirable that the breathing circuit apparatus include a drool trap which prevents the user's saliva from contaminating the medicament-containing liquid which is used to generate the aerosolized medicament. It would also be advantageous if the breathing circuit apparatus has an inlet which could be utilized for replenishing the liquid in the nebulizer device without interruption of use or monitoring breathing pressure in the breathing circuit apparatus while in use. The inlet could further be utilized as a sensing port for monitoring breathing cycles and pressures generated by the user/patient to control the timing of the flow of aerosolizing gas to the nebulizer device to occur during only pre-selected intervals during the user/patient's breathing cycles. The present invention satisfies these needs and provides these advantages.

### OBJECTS AND SUMMARY OF THE INVENTION

An object of the present invention is to provide a new and improved breathing circuit apparatus adapted for use in combination with a nebulizer device operative to generate an aerosol so that a user can inhale the aerosol through an opening, i.e. nose and/or mouth, in the user's respiratory system and into the user's lungs and thereafter exhaling exhalation gas from the opening in the user's respiratory system, through the breathing circuit apparatus and into ambient environment.

Another object of the present invention is to provide a breathing circuit apparatus in combination with a nebulizer device that creates a positive back-pressure on the lungs when the user exhales through the breathing circuit apparatus which, in turns, results in a desirable effect of prolonged expansion of the alveoli of the lungs, thus improving absorption of the aerosol into the user's body.

Still another object of the present invention is to provide a breathing circuit apparatus with an adjustable outlet valve so that the amount of positive back-pressure on the lungs can be regulated.

Yet another object of the present invention is to provide a breathing circuit apparatus with a nebulizer device that minimizes loss of aerosolized medicament into the ambient air, thus minimizing waste of the medication contained in the aerosol.

Yet a still further object of the present invention is to provide a breathing circuit apparatus with a nebulizer device that better enables a health care professional to provide a greater density of aerosolized medication for the patient per liter of air inhaled.

A still further object of the present invention is to provide a breathing circuit apparatus with a nebulizer device having a mouthpiece structure.

Yet another object of the present invention is to provide a breathing circuit apparatus with a nebulizer device having a drool trap which prevents the user's saliva from contaminating the medicament-containing liquid which is used to generate the aerosolized medicament.

A still further object of the present invention is to provide a breathing circuit apparatus having an inlet which could be utilized for a variety of reasons such as replenishing the liquid in the nebulizer without interruption of use and monitoring breathing pressure in the nebulizer while in use.

Yet another object of the present invention is to provide a breathing circuit apparatus with a nebulizer device having an inlet which could be utilized as a sensing port for monitoring breathing cycles and pressures generated by the user/patient to control the timing of the flow of aerosolizing gas to the breathing circuit apparatus to occur during only pre-selected intervals during the user/patient's breathing cycles.

Accordingly, a breathing circuit apparatus of the present invention is hereinafter described. In combination with a nebulizer device operative to generate an aerosol, the breathing circuit apparatus of the present invention is adapted for use by a user for inhaling the aerosol through an opening, i.e. nose and/or mouth, in a respiratory system of the user and into the user's lungs and thereafter exhaling exhalation gas from the nose and/or mouth of the user and through the breathing circuit apparatus. In its broadest form, the breathing circuit apparatus includes a container, an inlet valve, and outlet valve and a user connection. The container defines an interior chamber therein and is coupled to the nebulizer device. The container has an inlet orifice formed therethrough to provide fluid communication between the interior chamber and the nebulizer device. The interior chamber is sized and adapted to receive the aerosol generated by the nebulizer device through the inlet orifice.

The inlet valve is connected to the container and including an air inlet port for one-way air flow of ambient air disposed exteriorly of the container into the interior chamber. The inlet valve is operative to move between a closed condition to prevent the ambient air from entering into the interior chamber and an opened condition to permit the ambient air to enter into the interior chamber.

The outlet valve is connected to the container and includes a gas exhalation port for one-way gas flow of the exhalation gas from the user's respiratory system after inhalation. The outlet valve is operative to move between a closed state to prevent exhalation gas from exiting the interior chamber and an opened state to permit exhalation gas to exit from the interior chamber.

The user connection port is operative to be disposed between and in fluid communication with the interior chamber and the opening into the user's respiratory system when the user inhales and exhales. As the user inhales the aerosol from the interior chamber, the inlet valve is in the opened condition while simultaneously therewith the outlet valve is in the closed state so that the aerosol is inhaled into the lungs of the user without loss of the aerosol to the exterior ambient air. As the user exhales the exhalation gas from the user's respiratory system with at least a threshold amount of exhalation gas pressure and through the user connection port, the inlet valve is in the closed condition while simultaneously therewith the outlet valve is in the opened state to allow the exhalation gas to exit the interior chamber and discharge into the exterior ambient air. The threshold amount of exhalation gas pressure creates a positive back-pressure on the user's lungs.

The breathing circuit apparatus of the present invention includes a downdraft tube extending within the interior chamber to define a downdraft duct. The downdraft tube has a first end connected to the container which surrounds the air inlet port of the inlet valve and a free second end which is disposed opposite of the first end. When exterior ambient air enters through the air inlet port at the first end when the inlet valve is in the opened condition, the ambient air flows through the downdraft duct to the free second end.

The breathing circuit apparatus of the present invention also includes a deflector member that is disposed within the interior chamber and interposed between the free second end of the downdraft tube and the inlet orifice. The deflector member is positioned in a spaced apart relationship from the free second end of the downdraft tube and the inlet orifice. The deflector member and the inlet orifice are spaced apart from one another at a distance selected from a range between approximately 0.019 millimeters and 0.036 millimeters.

The breathing circuit apparatus of the present invention includes a plurality of spacers. The plurality of spacers interconnect the free second end of the downdraft tube and the deflector member and define spacer openings between sequential ones of the spacers. When the ambient air enters the container through the inlet valve, the ambient air passes through the downdraft duct and outwardly therefrom through the spacer openings.

The container of the breathing circuit apparatus of the present invention can be either unitary in construction or can be formed of two sections, an upper container section and a lower container section releasably connected to the upper container section. The upper container section includes a lower rim portion having a plurality of channels formed therein. The lower container section includes an upper rim portion having a plurality of dogs projecting radially outwardly therefrom. Respective ones of the dogs and the channels are sized and adapted for matable engagement with each other so that the upper container section and the lower container section can be releasably connected together in a fluid tight relation to form a unitary container.

The container includes an outlet conduit assembly having a conduit stem connected to and extending outwardly from an outer container wall of the container to form a conduit stem region of the interior chamber and a mouthpiece structure with a cross-member. A first mouthpiece end of the mouthpiece structure is sized and adapted to be slidably received into the conduit stem region. The outlet conduit assembly also includes a drool trap formed within the conduit stem region. The container also includes an inlet which is formed therethrough to provide fluid communication into the interior chamber. A plug sized and adapted to be removably received by the inlet.

Although it is preferred that the breathing circuit apparatus of the present invention be used in combination with the nebulizer device, the breathing circuit apparatus can also stand alone without the nebulizer device. Thus, the breathing circuit apparatus of the present invention may be used in other applications.

These and other objects of the present invention will become more readily appreciated and understood from consideration of the following detailed description of the exemplary embodiments of the present invention when taken in conjunction with the accompanying drawings, in which:

### BRIEF DESCRIPTION OF THE DRAWINGS

Figure 1 is a side elevational view in cross-section of a prior art nebulizer;
Figure 2 is a perspective view of a first exemplary embodiment of a breathing circuit apparatus of the present invention partially broken away and illustrated in combination with a nebulizer device shown operatively coupled to a pressurized aerosolizing gas source and a medicament source with a user/patient breathing into and from the breathing circuit apparatus;
Figure 3 is an exploded side view in cross-section of the breathing circuit apparatus of a preferred embodiment of the present invention that incorporates a nebulizer device as an integral portion thereof;
Figure 4 is a side view in cross-section of the breathing circuit apparatus of a preferred embodiment of the present invention showing an inlet valve having an air inlet port being in an opened condition and an outlet valve having an outlet port being in a closed state;
Figure 5 is a side view in cross-section of the breathing circuit apparatus of a preferred embodiment of the present invention showing the inlet valve with its air inlet port being in a closed condition and an outlet valve with its outlet port being in an opened state;
Figure 6 is an enlarged fragmentary side view in cross-section of the nebulizer device disposed within an interior chamber and positioned in a facially-opposing, spaced-apart, parallel relationship with a deflector member;
Figure 7 is a top plan view of a lower section of the breathing circuit apparatus of a preferred embodiment of the present invention taken along line 7 - 7 of Figure 3 and showing a plurality of dogs;
Figure 8 is a fragmentary side view in elevation of the lower section of the breathing circuit apparatus of a preferred embodiment of the present invention showing the plurality of dogs of Figure 7;
Figure 9 is a perspective view of a mouthpiece structure;
Figure 10 is a top plan view of the mouthpiece structure shown in Figure 9;
Figure 11 is a fragmentary side view partially in cross-section of a cross-member of the mouthpiece structure taken along line 11 - 11 in Figure 10; and
Figure 12 is a perspective view of a second exemplary embodiment of a breathing circuit apparatus of a preferred embodiment of the present invention shown with a diagrammatic nebulizer and a diagrammatic pressure transducer and monitor.

### DETAILED DESCRIPTION OF THE EXEMPLARY EMBODIMENTS

A breathing circuit apparatus of the present invention used in combination with a nebulizer device which produces aerosol is adapted for use by a user for inhaling the aerosol through an opening, i.e. nose and/or mouth in a respiratory system of the user and into the user's lungs and thereafter exhaling exhalation gas from the opening in the user's respiratory system and through the breathing circuit apparatus. One of ordinary skill in the art would appreciate that many different types of nebulizer devices exist that produce an aerosol for many different types of applications. Furthermore, a skilled artisan would appreciate that nebulizer devices are often employed in the medical industry to produce a medicament-containing aerosol from a liquid medicament for inhalation by a user/patient. As the description proceeds, the skilled artisan would appreciate that the breathing circuit apparatus of the present invention can be utilized in combination with any type of nebulizer device that produces an aerosol regardless if the aerosol produced contains medicament. Additionally, the breathing circuit apparatus of the present invention can either incorporate the nebulizer device into its structure as a unitary construction or be utilized separately from the nebulizer device but operatively coupled thereto. Only by way of example and not of limitation, the breathing circuit apparatus of the present invention is described and illustrated hereinafter with components of a prior art nebulizer discussed hereinabove and understood by one of ordinary skill in the art. Further discussion of these prior art components is deemed unnecessary.

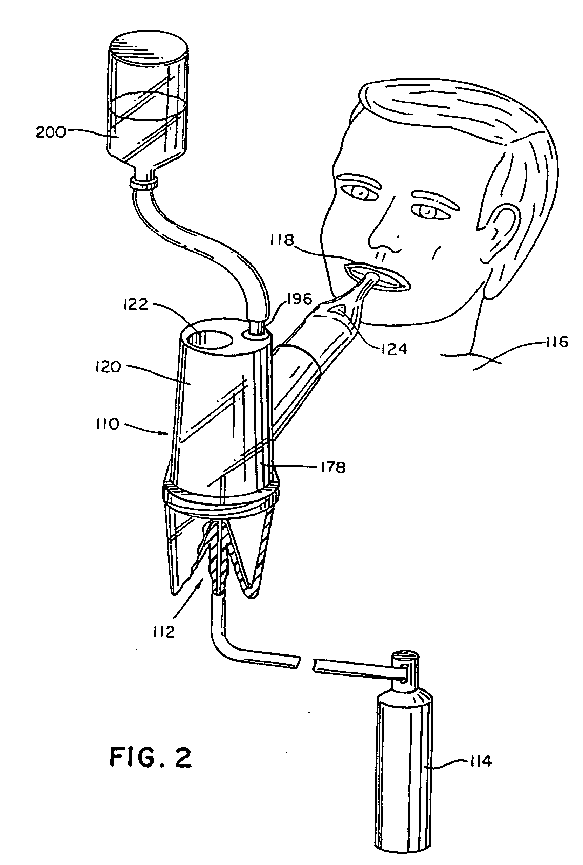

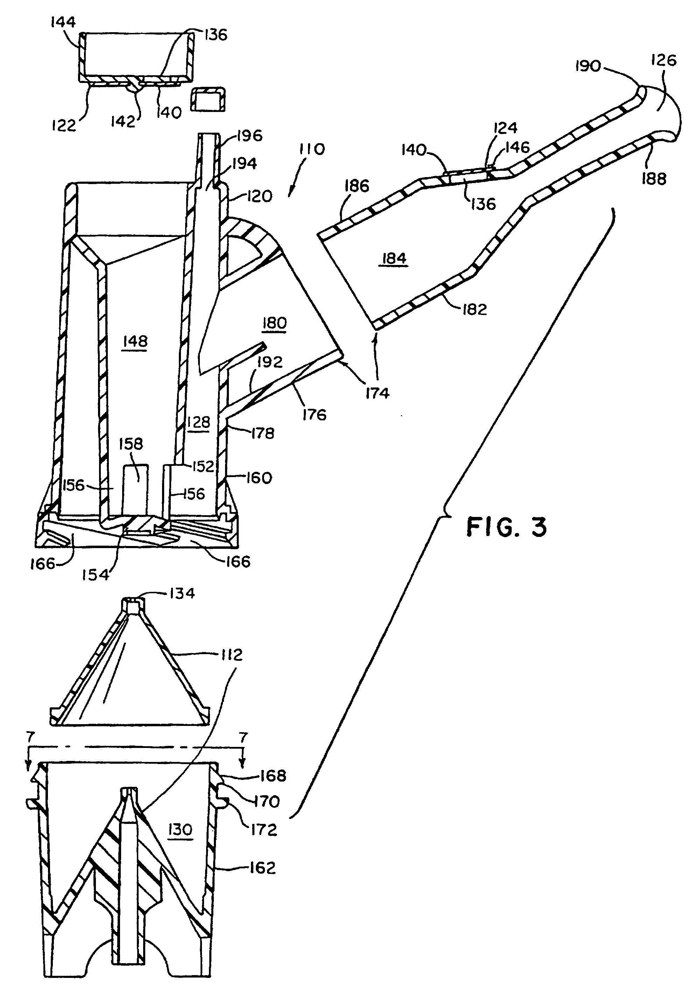

A first exemplary embodiment of a breathing circuit apparatus 110 of the present invention is generally introduced in Figures 2 - 11. With reference to Figures 2 and 3, a first exemplary embodiment of breathing circuit apparatus 110 is used in combination with a nebulizer device 112 which is formed as a unitary construction with breathing circuit apparatus 110 and is operably coupled to an aerosolizing pressurized gas source 114. Nebulizer device 112 is operative to generate an aerosol at gas pressures of from about 137895.2 to 344738.0 N/m² (20 psi to 50 psi) at a flow rate of 6 to 8 liters per minute. Breathing circuit apparatus 110 is adapted for use by a user 116 for inhaling the aerosol through an opening 118, the user's mouth, in a respiratory system of user 116 and into the user's lungs and thereafter exhaling exhalation gas from opening 118 in the user's respiratory system and through breathing circuit apparatus 110. Breathing circuit apparatus comprises a container 120, an inlet valve 122, an outlet valve 124 and a user connection port 126.

Container 120, generally cylindrical in shape, defines an interior chamber 128 therein. For the first embodiment of breathing circuit apparatus 110 interior chamber 128 includes a reservoir region 130 which holds the liquid to be nebulized and an aerosol region 132 for receiving the aerosol after the liquid becomes nebulized. Container 120 is operatively coupled to nebulizer device 112 having an inlet orifice 134 which provides fluid communication between interior chamber 128 and nebulizer device 112. At least reservoir region 130 of interior chamber 128 is sized and adapted to receive the aerosol generated by nebulizer device 112 through inlet orifice 134.

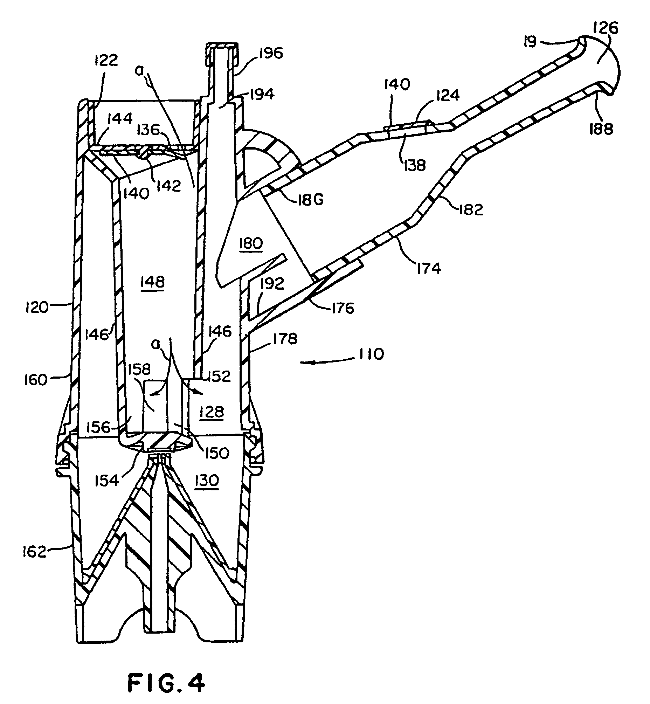

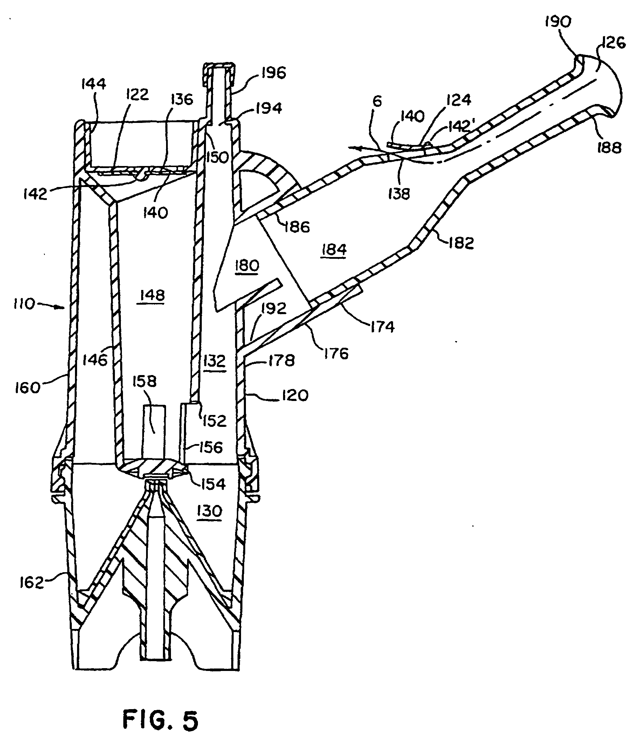

As best shown in Figures 3 - 6, inlet valve 122 is connected to container 120 and includes an air inlet port 136 which provides for one-way air flow of ambient air (represented by solid arrow "a" in Figure 4) disposed exteriorly of container 120 into interior chamber 128. Inlet valve 122 is operative to move between a closed condition (Figure 5) to prevent the ambient air "a" from entering into interior chamber 128 and an opened condition (Figure 4) to permit the ambient air "a" to enter into interior chamber 128. Outlet valve 124 is connected to container 120 and includes a gas exhalation port 138 for one-way gas flow of the exhalation gas (represented by dashed arrow "b" in Figure 5) from the user's respiratory system after inhalation by user 116. Outlet valve 124 is operative to move between a closed state (Figure 4) to prevent exhalation gas "b" from exiting interior chamber 128 and an opened state to permit exhalation gas "b" to exit from interior chamber 128.

User connection port 126 is operative to be disposed between and in fluid communication with interior chamber 128 and the opening 118, i.e. the user's nose and/or mouth, into the user's respiratory system when user 116 inhales and exhales. As user 116 inhales the aerosol from interior chamber 128, inlet valve 122 is in the opened condition while simultaneously therewith outlet valve 124 is in the closed state as shown in Figure 4. When this occurs, the aerosol is inhaled into the lungs of user 116 without loss of the aerosol to the exterior ambient air. As user 116 exhales the exhalation gas "b" from the user's respiratory system and through user connection port 126 with at least a threshold amount of exhalation gas pressure, thereby creating a positive back-pressure on the user's lungs, inlet valve 122 is in the closed condition while simultaneously therewith outlet valve 124 is in the opened state to allow the exhalation gas "b" to exit interior chamber 128 and discharge into the exterior ambient air as shown in Figure 5.

Again with reference to Figure 3, inlet valve 122 is normally disposed in the closed condition and, likewise, outlet valve 124 is normally disposed in the closed state. Specifically, inlet valve 122 is resiliently biased in the normally closed condition and outlet valve 124 is resiliently biased in the normally closed state. It is preferred that each of inlet valve 122 and outlet valve 124 is a flap valve having a flap 140 which is fabricated from a stiff yet resilient material normally used for flap valves such as rubber or plastic. Although not by way of limitation, rivet 142 retains flap 140 onto a valve housing 144 for inlet valve 122 while rivet 142' retains flap 140 over gas exhalation port 138 of outlet valve 124. Depending upon thickness and selection of the flap material, the amount of pressure required to open inlet valve 122 and outlet valve 124 can vary. Thus, by way of example, the exhalation gas pressure can be varied by changing the resistance to flow described, thus enabling more effective usage by the patient of medicament-containing aerosol, because of the resultant increase in the time required for patient exhalation. The efficacy of the aerosolized medicament is improved by this longer exposure to the patient's expanded lungs. Typically, resistance to exhalation should be in the range of from about 5 cm to 20 cm of water pressure during normal exhalation.

As best shown in Figures 3 - 5, breathing circuit apparatus 110 includes a downdraft tube 146 which extends within interior chamber 128 to define a downdraft duct 148. Downdraft tube 146 includes a first end 150 and a free second end 152. First end 150 is connected to container 120 and surrounds air inlet port 136 of inlet valve 122. Free second end 152 is disposed opposite of first end 150 so that, as illustrated in Figure 4, exterior ambient air "a" enters through air inlet port 136 at first end 150 when inlet valve 122 is in the opened condition and flows through downdraft duct 148 to free second end 152.

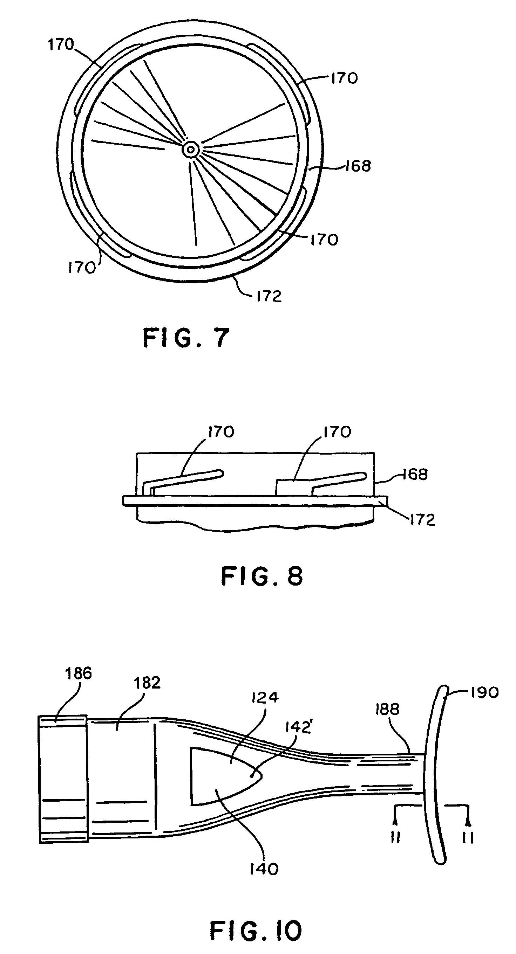

In Figures 3 - 6, breathing circuit apparatus 110 includes a deflector member 154 which is disposed within interior chamber 128 and is interposed between free second end 152 of downdraft tube 146 and inlet orifice 134. Deflector member 154 is positioned in a spaced apart relationship from free second end 152 of downdraft tube 146 and inlet orifice 134. For the first exemplary embodiment of the breathing circuit apparatus of the present invention, deflector member 154 and inlet orifice 134 are spaced apart from one another at a distance "d" shown in Figure 6 which is selected from a range between approximately 0.019 millimeters and 0.036 millimeters. To best describe the orientation of inlet orifice 134 relative to deflector member 154, inlet orifice 134 defines a first imaginary plane "P₁" while deflector member 154 defines a second imaginary plane "P₂". Preferably, first imaginary plane "P₁" and second imaginary plane "P₂" are facially opposed to and parallel with one another.

Again, with reference to Figures 2 - 6, breathing circuit apparatus 110 includes a plurality of spacers 156. The plurality of spacers 156 interconnect free second end 152 of downdraft tube 146 and deflector member 154. Additionally, the plurality of spacers define spacer openings 158 which are located between sequential ones of spacers 156. As best illustrated in Figure 4, when the ambient air "a" enters container 120 through inlet valve 122, the ambient air "a" passes through downdraft duct 148 and outwardly therefrom through spacer openings 158.

Although not by way of limitation, container 120 comprises an upper container section 160 and a lower container section 162 which is releasably connected to upper container section 160. Particularly as shown in Figures 3, 7 and 8, upper container section 160 includes a lower rim portion 164 having a plurality of channels 166 formed therein. Also, lower container section 162 includes an upper rim portion 168 having a plurality of dogs 170 projecting radially outwardly from upper rim portion 168. As commonly known in the industry, respective ones of dogs 170 and channels 166 are sized and adapted for matable engagement with each other so that upper container section 160 and lower container section 162 can be releasably connected together in a fluid tight relation to form a unitary container. An annular rim element 172 extends circumferentially about and projects radially outwardly from upper rim portion 168 of lower container section 162.

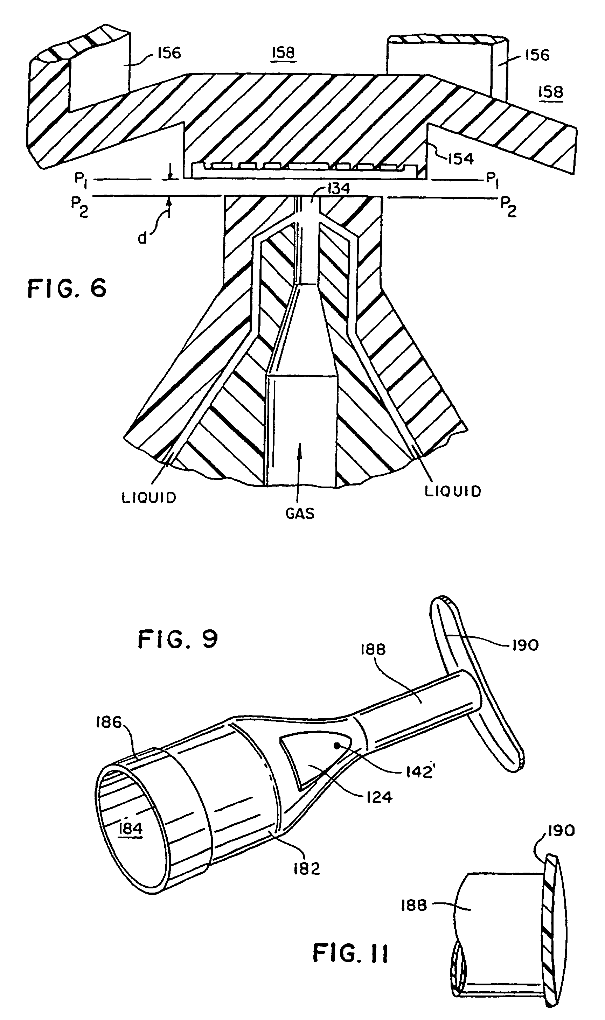

As shown in Figures 3 - 5, container 120 further includes an outlet conduit assembly 174 that has a conduit stem 176. Conduit stem 176 is connected to and extends outwardly from an outer container wall 178 of container 120 in an upwardly direction relative to lower container section 162. Conduit stem 176 defines a conduit stem region 180 of interior chamber 128. Preferably, conduit stem 176 is generally configured in a cylindrical shape. As best shown in Figures 9 - 11, outlet conduit assembly 174 includes a mouthpiece structure 182 that defines a mouthpiece region 184 of interior chamber 128. It is preferred that mouthpiece structure 182 incorporate outlet valve 124 with its accompanying gas exhalation port 138 thereinto as illustrated by the appropriate figures. Mouthpiece structure 182 has a first mouthpiece end 186 sized and adapted to be slidably received into conduit stem region 180 and a second mouthpiece end 188 that is disposed opposite first mouthpiece end 186 and is connected to a cross-member 190.

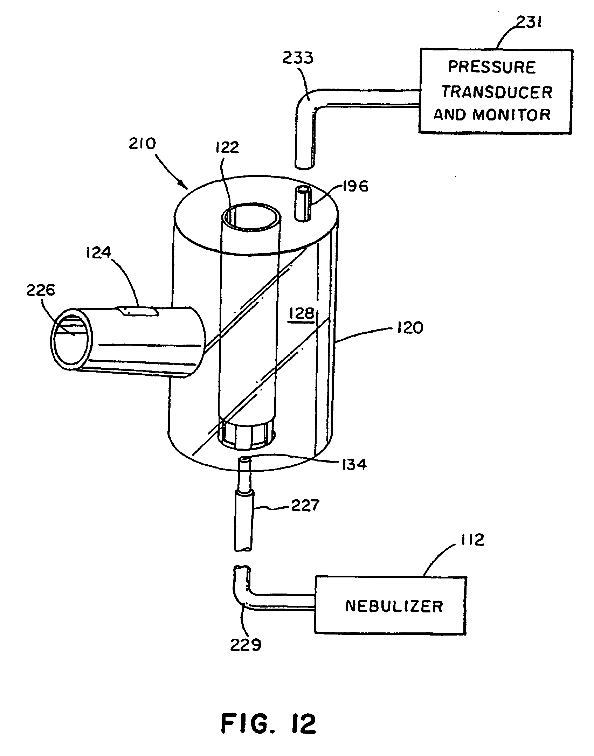

Cross-member 190 extends transversely to second mouthpiece end 188. For the first exemplary embodiment of the breathing circuit apparatus of the present invention, cross-member 190 includes user connector port 126. By example only and not limitation, cross-member 190 is arcuate in cross-section and forms a concavity as best illustrated in Figures 10 and 11. When breathing circuit apparatus 110 is operative, cross-member 190 is disposed above conduit stem 176 when first mouthpiece end 186 is slidably received by conduit stem region 180.

Further, outlet conduit assembly 174 includes a drool trap 192. Drool trap 192 is formed within conduit stem region 180 by a portion of outer container wall 178 and a bottom portion of conduit stem 176. Since breathing circuit apparatus 110 of the present invention is designed so that user 116 continuously inhales therefrom and exhales thereinto, user 116 may tend to drool into breathing circuit apparatus 110. Drool trap 192 entraps any drool emanating from the user's mouth and prevents the same from contaminating the medicament-containing liquid held in reservoir region 130 of interior chamber 128.

Additionally, as shown in Figures 2 - 6, container 120 includes an inlet 194 formed through container 120 to provide fluid communication into interior chamber 128. An inlet tube 196 connects inlet 194 and projects outwardly from container 120. A plug 198 in a form of a cap is sized and adapted to be removably received by either inlet 194 or inlet tube 196 so that when plug 198 is received by inlet 194 or inlet tube 196, fluid communication into interior chamber 128 through inlet 194 or inlet tube 196 is prevented. As illustrated in Figure 2, one use of inlet 194 is to allow a source of liquid 200 to be coupled in fluid communication with interior chamber 128 so that the liquid can flow into container 120 without interrupting the operation of the breathing circuit apparatus 110 in combination with nebulizer device 112.

A second exemplary embodiment of a breathing circuit apparatus 210 of the present invention is shown in Figure 12. This second exemplary embodiment of breathing circuit apparatus is not integrated as a unitary construction with nebulizer device 112 but stands alone. Breathing circuit apparatus 210 includes container 120, inlet valve 122, outlet valve 124 and an opened port 226. Opened port 225 is formed into container 120 and is in fluid communication with interior chamber 128 and the ambient air environment. Container 120 has inlet orifice 134 formed therethrough. Inlet orifice 134 sized and adapted to conduct a nebulized liquid into interior chamber 128 from nebulizer device 112 which is located remotely from breathing circuit apparatus 210. An inlet orifice tube 227 is coupled in fluid communication with inlet orifice 134 so that aerosol generated by nebulizer device 112 can be conveyed through a nebulizer conduit 229 and into container 120.

A pressure transducer/monitor 231 is coupled in fluid communication with inlet tube 196, utilized as a sensing port, by a monitor conduit 233. With this arrangement, events of pressures occurring in interior chamber 128 as the user inhales and exhales can be monitored, if desired. This information obtained by pressure transducer/monitor 231 can be utilized, for example, to electronically control and electrically operate electrical inlet and outlet valves as well as to electronically control the timing and amounts of aerosol. This sensing port is adapted for monitoring breathing cycles and pressures generated by the user/patient and can control the timing of the flow of aerosolizing gas to the breathing circuit apparatus which can be caused to occur during only pre-selected intervals during the user/patient's breathing cycles.

It is appreciated that the breathing circuit apparatus of the present invention used in combination with the nebulizer can be employed by the user to both inhale the aerosol through his/her nose and/or mouth and exhale exhalation gas through the breathing circuit apparatus and into ambient environment. The outlet valve on the container of the breathing circuit apparatus creates a positive back-pressure on the user's lungs when the user exhales through the breathing circuit apparatus. Back-pressure on the user's lungs is desirable because such back-pressure causes prolonged expansion of the alveoli of the lungs. Prolonged expansion of the alveoli of the lungs results in improved absorption of the medicament-containing aerosol into the user's body. Also, the outlet valve can be adjustable (not shown) so that the amount of positive back-pressure on the lungs can be regulated. With the inlet and outlet valves being normally closed and the breathing circuit apparatus designed to be a "breath-through" device in continuous contact with the user's nose and/or mouth, a minimum amount of aerosolized medicament is lost into the ambient air. As a result, waste of the medication contained in the aerosol is minimized. Furthermore, with minimum waste of medication, health care provider is positioned to better predict medicament dosage requirements for the user/patient.

The breathing circuit apparatus includes several structural features that benefit the user/patient. The mouthpiece structure is designed so that the user/patient can continuously breath through the breathing circuit apparatus. The drool trap prevents the user's saliva from contaminating the medicament-containing liquid. The inlet could be utilized for a variety of reasons such as replenishing the liquid in the nebulizer without interruption of use and monitoring breathing pressure in the nebulizer while in use. Specifically, the inlet can be utilized as a sensing port for monitoring breathing cycles and pressures generated by the user/patient to control the timing of the flow of aerosolizing gas to the breathing circuit apparatus to occur during only pre-selected intervals during the user/patient's breathing cycles.

## Claims

1. A breathing circuit apparatus (110) for use in combination with a nebulizer device (112) operative to generate an aerosol, the breathing circuit apparatus (110) being adapted for use by a user (116) for inhaling the aerosol through an opening (118) in a respiratory system of the user (116) and into the user's lungs and thereafter exhaling exhalation gas from the opening (118) in the user's respiratory system and through the breathing circuit apparatus (110), the breathing circuit apparatus (110) including;
(a) a breathing circuit container (120) having an interior chamber (128) for receiving the aerosol from nebulizer device (112) with an inlet orifice (134) from the nebulizer (112) extending into the interior chamber (128) to provide fluid communication between the interior chamber (128) and the nebulizer device (112) and being sized and adapted to receive the aerosol generated by the nebulizer device (112) through the inlet orifice (134);
(b) an inlet valve (122) connected to the breathing circuit breathing circuit container (120) and including an air inlet port (136) for one-way air flow of ambient air disposed exteriorly of the breathing circuit container (120) into the interior chamber (128), the inlet valve (122) operative to move between a closed condition to prevent the ambient air from entering into the interior chamber (128) and an opened condition to permit the ambient air to enter into the interior chamber (128);
(c) a downdraft tube (146) extending within the interior chamber (128) of the breathing circuit container (120) to define a downdraft duct (148) and having a first end (150) connected to the breathing circuit container (120) and surrounding the air inlet port (136) of the inlet valve (122) and a second end (152) disposed opposite of the first end (150) so that exterior ambient air enters through the air inlet port (136) at the first end (150) when the inlet valve (122) is in the opened condition and flows through the downdraft duct (148) to the second end (152);
(d) an output conduit assembly (174) for communicating between the respiratory system of the user (116) and the breathing circuit container (120), including
(1) an outlet valve (124) for communicating with the breathing circuit container (120) and including a gas exhalation port (138) for one-way gas flow of the exhalation gas from the user's respiratory system after inhalation, the outlet valve (124) operative to move between a closed state and an opened state to permit exhalation gas to exit from the interior chamber (128); and
(2) a user connection port (126) for communicating with the breathing circuit container (120) and operative to be disposed between and in fluid communication with the interior chamber (128) and the opening into the user's respiratory system when the user (116) inhales and exhales whereby,
as the user (116) inhales the aerosol from the interior chamber (128), the inlet valve (122) is in the opened condition while simultaneously therewith the outlet valve (124) is in the closed state so that the aerosol is inhaled into the lungs of the user (116) without loss of the aerosol to the exterior ambient air and,
as the user (116) exhales the exhalation gas from the user's respiratory system and through the user connection port (126), the inlet valve (122) is in the closed condition while simultaneously therewith the outlet valve (124) is in the opened state to allow the exhalation gas to exit the interior chamber (128) and discharge into the exterior ambient air;
the breathing circuit apparatus (110) being **characterized by**:
(e) a conduit stem (176) extending outwardly from an outer container wall (178) of the breathing circuit container (120) for receiving and connecting the output conduit assembly (174) with the breathing circuit container (120) and including a drool trap (192) formed within the conduit stem (176) by a portion of the outer container wall (178) and a bottom portion of the conduit stem (176) for entrapping drool from the user's mouth and preventing drool from entering the interior chamber (128).

2. The breathing circuit apparatus according to claim 1, further **characterized in that** the second end (152) is free of attachment to the breathing circuit container (120) and includes a deflector member (154) disposed within the interior chamber (128) and interposed between the free second end (152) of the downdraft tube (146) and the inlet orifice (134), the deflector member (154) positioned in a spaced apart relationship from the free second end (152) of the downdraft tube (146) and the inlet orifice (134).

3. The breathing circuit apparatus (110) according to claim 2, further **characterized in that** the deflector member (154) and the inlet orifice (134) are spaced apart from one another at a distance selected from a range between approximately 0.019 millimeters and 0.036 millimeters.

4. The breathing circuit apparatus (110) according to claim 2, further **characterized in that** the inlet orifice (134) defines a first imaginary plane and the deflector member (154) defines a second imaginary plane, and the first and second imaginary planes are facially opposed to and parallel with one another.

5. The breathing circuit apparatus (110) according to claim 2, further **characterized in that** a plurality of spacers (156) interconnect the free second end (152) of the downdraft tube (146) and the deflector member (154) and define spacer openings (158) between sequential ones of the spacers (156) so that when the ambient air enters the breathing circuit container (120) through the inlet valve (122), the ambient air passes through the downdraft duct (148) and outwardly therefrom through the spacer openings (158).

6. The breathing circuit apparatus (110) according to claim 1, in which the breathing circuit apparatus (110) and the nebulizer (112) are further **characterized in that** a nebulizer container (162), connectable to a lower end of the breathing circuit container (120) and containing the nebulizer device (112), provides passage of the aerosol between the nebulizer (112) and the interior chamber (128).

7. The breathing circuit apparatus (110) according to claim 6, in which the breathing circuit apparatus (110) and the nebulizer (112) are further **characterized in that** the breathing circuit container (120) and the nebulizer container (160) are connectable so that the inlet orifice (134) from the nebulizer device (112) extends into the interior chamber (128) to provide passage of the aerosol between the nebulizer device (112) and the interior chamber (128) so that a first imaginary plane, defined by the inlet orifice (134), and a second imaginary plane, defined by the deflector member (154), are facially opposed to and parallel with one another.

8. The breathing circuit apparatus (110) according to claim 7, in which the breathing circuit apparatus (110) and the nebulizer (112) are further **characterized in that** the nebulizer device (112) has an upwardly oriented first conical member having a circumferential perimeter at a lower end connected to an interior wall of the nebulizer container (160) to provide a liquid reservoir (130) between an upper surface of the first conical member and the interior wall of the nebulizer container (160), and a lower central gas passage extending upwardly through the vertical centerline of the first conical member, and
a second conical member having a hollow form such that an inner surface of the second conical member and the upper surface of the first conical member form a liquid passage for drawing liquid from the liquid reservoir and upwardly between the first and second conical members to the upper end of the lower central gas passage in the first conical member, the second conical member including an upper central gas passage extending upward from the lower central gas passage and terminating in the inlet orifice (134) and being mechanically independent and removable from the nebulizer container (160), the first conical member and the breathing circuit apparatus (110).

## Patentansprüche

1. Atmungskreisapparat (110) zum Gebrauch in Verbindung mit einer Zerstäubungsvorrichtung (112), die wirksam ist, um ein Aerosol zu erzeugen, wobei der Atmungskreisapparat (110) ausgebildet ist, um von einem Benutzer (116) verwendet zu werden, um das Aerosol durch eine Öffnung (118) in einem Atmungssystem des Benutzers (116) und in die Lungen des Benutzers einzuatmen und danach das Ausatmungsgas aus der Öffnung (118) in dem Atmungssystem des Benutzers und durch den Atmungskreisapparat (110) auszuatmen, wobei der Atmungskreisapparat (110) folgendes enthält:
(a) einen Atmungskreisbehälter (120), der eine innere Kammer (128) zum Empfangen des Aerosols von der Zerstäubervorrichtung (112) hat, wobei eine Einlaßöffnung (134) des Zerstäubers (112) sich in die innere Kammer (128) erstreckt, um eine Fluidverbindung zwischen der inneren Kammer (128) und der Zerstäubervorrichtung (112) bereitzustellen, und die so bemessen und ausgebildet ist, um das von der Zerstäubervorrichtung (112) erzeugte Aerosol durch die Einlaßöffnung (134) zu empfangen,
(b) ein Einlaßventil (122), das mit dem Atmungskreisbehälter (120) verbunden ist und eine Lufteinlaßöffnung (136) für einen Einwegluftstrom aus Umgebungsluft, die sich außerhalb des Atmungskreisbehälters (120) befindet, in die innere Kammer (128), wobei das Einlaßventil (122) wirksam ist, um sich zwischen einer Geschlossenstellung zum Verhindern, daß Umgebungsluft in die innere Kammer (128) eintritt, und einer Offenstellung zum Zulassen, daß die Umgebungsluft in die innere Kammer (128) eintritt, zu bewegen;
(c) ein Fallstromrohr (146), das sich innerhalb der inneren Kammer (128) des Atmungskreisbehälters (120) erstreckt, um einen Fallstromkanal (148) zu begrenzen, und ein mit dem Atmungskreisbehälter (120) verbundenes und die Lufteinlaßöffnung (136) des Einlaßventiles (122) umgebendes erstes Ende (150) und ein gegenüber dem ersten Ende (150) angeordnetes zweites Ende (152) hat, so daß äußere Umgebungsluft durch die Lufteinlaßöffnung (136) an dem ersten Ende (150) eintritt, wenn das Einlaßventil (122) in der Offenstellung ist, und durch den Fallstromkanal (148) zu dem zweiten Ende (152) strömt;
(d) eine Ausgangsleitungsanordnung (174), um eine Verbindung zwischen dem Atmungssystem des Benutzers (116) und dem Atmungskreisbehälter (120) herzustellen, die folgendes enthält:
(1) ein Auslaßventil (124), um eine Verbindung zu dem Atmungskreisbehälter (120) herzustellen und das eine Gasausatmungsöffnung (138) für eine Einweggasströmung des Ausatmungsgases von dem Atmungssystem des Benutzers nach der Einatmung enthält, wobei das Auslaßventil (124) wirksam ist, um sich zwischen einer Geschlossenstellung und einer Offenstellung zu bewegen, um dem Ausatmungsgas zu gestatten, aus der inneren Kammer (128) auszutreten; und
(2) eine Benutzeranschlußöffnung (126), um eine Verbindung zu dem Atmungskreisbehälter (120) herzustellen, und die wirksam ist, um zwischen und in Fluidverbindung mit der inneren Kammer (128) und der Öffnung in das Atmungssystem des Benutzers angeordnet zu werden, wenn der Benutzer (116) einatmet und ausatmet, wodurch, wenn der Benutzer (216) das Aerosol von der inneren Kammer (128) einatmet, das Einlaßventil (122) in der Offenstellung ist, während gleichzeitig damit das Auslaßventil (124) in der Geschlossenstellung ist, so daß das Aerosol in die Lungen des Benutzers (116) ohne Verlust des Aerosols an die äußere Umgebungsluft eingeatmet wird, und
wenn der Benutzer (116) das Ausatmungsgas aus dem Atmungssystem des Benutzers und durch die Benutzeranschlußöffnung (126) ausatmet, das Einlaßventil (122) in der Geschlossenstellung ist, während gleichzeitig damit das Auslaßventil (124) in der Offenstellung ist, um dem Ausatmungsgas zu gestatten, die innere Kammer (128) zu verlassen und in die äußere Umgebungsluft auszutreten;
wobei der Atmungskreisapparat (110) **gekennzeichnet ist durch**:
(e) einen Leitungsschaft (176), der sich nach außen von einer äußeren Behälterwand (178) des Atmungskreisbehälters (120) erstreckt, um die Ausgangsleitungsanordnung (174) aufzunehmen und sie mit dem Atmungskreisbehälter (120) zu verbinden, und einen Speichelfänger (192) enthält, der innerhalb des Leitungsschaftes (176) **durch** einen Teil der äußeren Behälterwand (178) und einem Bodenteil des Leitungsschaftes (176) gebildet ist, um Speichel aus dem Mund des Benutzers aufzufangen und zu verhindern, daß Speichel in die innere Kammer (128) eintritt.

2. Atmungskreisapparat gemäß Anspruch 1, ferner **dadurch gekennzeichnet, daß** das zweite Ende (152) keine feste Verbindung zu dem Atmungskreisbehälter (120) hat und ein Ablenkteil (154) enthält, das in der inneren Kammer (128) angeordnet ist und zwischen dem freien zweiten Ende (152) des Fallstromrohres (146) und der Einlaßöffnung (134) angeordnet ist, wobei das Ablenkteil (154) im Abstand von dem freien zweiten Ende (152) des Fallstromrohres (146) und der Einlaßöffnung (134) angeordnet ist.

3. Atmungskreisapparat (110) nach Anspruch 2, ferner **dadurch gekennzeichnet, daß** das Ablenkteil (154) und die Einlaßöffnung (134) um eine Strecke voneinander beabstandet sind, die aus einem Bereich zwischen ungefähr 0,019 mm und 0,036 mm ausgewählt ist.

4. Atmungskreisapparat (110) gemäß Anspruch 2, ferner **dadurch gekennzeichnet ist, daß** die Einlaßöffnung (134) eine erste gedachte Ebene begrenzt und das Ablenkteil (154) eine zweite gedachte Ebene begrenzt und die erste und zweite gedachte Ebene zueinander entgegengesetzt und parallel zueinander sind.

5. Atmungskreisapparat (110) nach Anspruch 2, ferner **dadurch gekennzeichnet, daß** eine Vielzahl von Distanzstücken (156) das freie zweite Ende (152) des Fallstromrohres (146) und das Ablenkteil (154) verbinden und Distanzöffnungen (158) zwischen aufeinanderfolgenden Distanzstücken (156) begrenzen, so daß, wenn die Umgebungsluft in den Atmungskreisbehälter (120) durch das Einlaßventil (122) eintritt, die Umgebungsluft durch den Fallstromkanal (148) hindurch und von dort nach außen durch die Distanzöffnungen (158) strömt.

6. Atmungskreisapparat (110) nach Anspruch 1, bei dem der Atmungskreisapparat (110) und der Zerstäuber (112) weiter **dadurch gekennzeichnet sind, daß** ein Zerstäuberbehälter (162), der mit einem unteren Ende des Atmungskreisbehälters (120) verbindbar ist und die Zerstäubungsvorrichtung (112) enthält, einen Durchgang für das Aerosol zwischen dem Zerstäuber (112) und der inneren Kammer (128) bereitstellt.

7. Atmungskreisapparat (110) nach Anspruch 6, bei dem der Atmungskreisapparat (110) und der Zerstäuber (112) weiter **dadurch gekennzeichnet sind, daß** der Atmungskreisbehälter (120) und der Zerstäuberbehälter (160) verbindbar sind, so daß die Einlaßöffnung (134) von der Zerstäubungsvorrichtung (112) sich in die innere Kammer (128) erstreckt, um einen Durchgang für das Aerosol zwischen der Zerstäubungsvorrichtung (112) und der inneren Kammer (128) bereitzustellen, so daß eine erste gedachte Ebene, die von der Einlaßöffnung (134) begrenzt wird und eine zweite gedachte Ebene, die von dem Ablenkteil (154) begrenzt wird, zueinander entgegengesetzt und parallel zueinander sind.

8. Atmungskreisapparat (110) nach Anspruch 7, bei dem der Atmungskreisapparat (110) und der Zerstäuber (112) weiter **dadurch gekennzeichnet sind, daß** die Zerstäubungsvorrichtung (112) ein nach oben gerichtetes erstes konisches Teil hat, von dem ein Umfang an einem unteren Ende mit einer inneren Wand des Zerstäuberbehälters (160) verbunden ist, um einen Flüssigkeitsspeicher (130) zwischen einer oberen Oberfläche des ersten konischen Teiles und der inneren Wand des Zerstäuberbehälters (160) und einen unteren zentralen Gasdurchgang zu schaffen, der sich nach oben durch die vertikale Mittellinie des ersten konischen Teiles erstreckt, und
ein zweites konisches Teil hat, das eine hohle Form hat, derart, daß eine innere Oberfläche des zweiten konischen Teiles und der oberen Oberfläche des ersten konischen Teiles einen Flüssigkeitsdurchgang zum Abziehen von Flüssigkeit aus dem Flüssigkeitsspeicher und nach oben zwischen dem ersten und zweiten konischen Teil zu dem oberen Ende des unteren zentralen Gasdurchganges in dem ersten konischen Teil bildet, wobei das zweite konische Teil einen oberen zentralen Gasdurchgang enthält, der sich nach oben von dem unteren zentralen Gasdurchgang aus erstreckt und in der Einlaßöffnung (134) endet und mechanisch unabhängig und entfernbar von dem Zerstäuberbehälter (160), dem ersten konischen Teil und dem Atmungskreisapparat (110) ist.

## Revendications

1. Appareil à circuit respiratoire (110) pour une utilisation avec un dispositif de nébuliseur (112) en mesure de générer un aérosol, ledit appareil à circuit respiratoire (110) étant adapté pour être utilisé par un utilisateur (116) pour inhaler l'aérosol au travers d'une ouverture (118) dans le système respiratoire de l'utilisateur (116) et dans les poumons de l'utilisateur, puis exhaler le gaz d'exhalation à partir de l'ouverture (118) dans le système respiratoire de l'utilisateur et au travers de l'appareil à circuit respiratoire (110), ledit appareil à circuit respiratoire (110) comprenant :
(a) un conteneur de circuit respiratoire (120) ayant une chambre intérieure (128) pour recevoir l'aérosol à partir du dispositif de nébuliseur (112) avec un orifice d'entrée (134) depuis le nébuliseur (112) qui s'étend dans la chambre intérieure (128) pour assurer la communication de fluide entre la chambre intérieure (128) et le dispositif de nébuliseur (112) et étant dimensionné et adapté pour recevoir l'aérosol généré par le dispositif de nébuliseur (112) au travers de l'orifice d'entrée (134) ;
(b) un clapet d'entrée (122) relié au conteneur de circuit respiratoire (120) et comprenant un orifice d'entrée d'air (136) pour le flux d'air unilatéral d'air ambiant placé à l'extérieur du conteneur de circuit respiratoire (120) dans la chambre intérieure (128), le clapet d'entrée (122) étant capable de se déplacer entre un état fermé pour empêcher l'air ambiant de pénétrer dans la chambre intérieure (128) et un état ouvert pour permettre à l'air ambiant de pénétrer dans la chambre intérieure (128).
(c) un tube de courant descendant (146) qui s'étend à l'intérieur de la chambre intérieure (128) du conteneur de circuit respiratoire (120) pour définir un conduit de courant descendant (148) et ayant une première extrémité (150) reliée au conteneur de circuit respiratoire (120) et entourant l'orifice d'entrée d'air (136) du clapet d'entrée (122) et une seconde extrémité (152) disposée à l'opposé de la première extrémité (150) de telle sorte que l'air ambiant extérieur pénètre par l'orifice d'entrée d'air (136) à la première extrémité (150) quand le clapet d'entrée (122) est dans l'état ouvert et s'écoule par le conduit de courant descendant (148) vers la seconde extrémité (152) ;
(d) un ensemble de conduit de sortie (174) pour communiquer entre le système respiratoire de l'utilisateur (116) et le conteneur de circuit respiratoire (120), comprenant :
(1) un clapet de sortie (124) pour communiquer avec le conteneur de circuit respiratoire (120) et comprenant un orifice d'exhalation de gaz (138) pour l'écoulement unilatéral du gaz d'exhalation depuis le système respiratoire de l'utilisateur après l'inhalation, le clapet de sortie (124) pouvant se déplacer entre un état fermé et un état ouvert pour permettre au gaz d'exhalation de sortir à partir de la chambre intérieure (128) ; et
(2) un orifice de raccordement pour l'utilisateur (126) pour communiquer avec le conteneur de circuit respiratoire (120) et pouvant être placé entre et en communication de fluide avec la chambre intérieure (128) et l'ouverture dans le système respiratoire de l'utilisateur quand l'utilisateur (116) inhale et exhale dans lequel,
quand l'utilisateur (116) inhale l'aérosol de la chambre intérieure (128), le clapet d'entrée (122) est dans l'état ouvert alors que simultanément le clapet de sortie (124) est dans l'état fermé de telle sorte que l'aérosol est inhalé dans les poumons de l'utilisateur (116) sans perte de l'aérosol vers l'air ambiant extérieur, et
quand l'utilisateur (116) exhale le gaz d'exhalation du système respiratoire de l'utilisateur et par l'orifice de raccordement de l'utilisateur (126), le clapet d'entrée (122) est dans l'état fermé alors que simultanément le clapet de sortie (124) est dans l'état ouvert pour permettre au gaz d'exhalation de sortir de la chambre intérieure (128) et de s'évacuer vers l'air ambiant extérieur ;
l'appareil à circuit respiratoire (110) étant **caractérisé par** :
(e) une tige de conduit (176) qui s'étend vers l'extérieur depuis une paroi extérieure de conteneur (178) du conteneur de circuit respiratoire (120) pour recevoir et raccorder l'ensemble du conduit de sortie (174) avec le conteneur de circuit respiratoire (120) et comprenant un collecteur de salive (192) formé à l'intérieur de la tige de conduit (176) par une partie de la paroi extérieure du conteneur (178) et une partie inférieure de la tige du conduit (176) pour recueillir la salive de la bouche de l'utilisateur et empêcher la salive de pénétrer dans la chambre intérieure (128).

2. Appareil à circuit respiratoire selon la revendication 1, également **caractérisé en ce que** la seconde extrémité (152) est exempte de fixation sur le conteneur de circuit respiratoire (120) et comprend un élément déflecteur (154) placé à l'intérieur de la chambre intérieure (128) et interposé entre la seconde extrémité libre (152) du tube de courant descendant (146) et l'orifice d'entrée (134), l'élément déflecteur (154) étant positionné en relation espacée par rapport à la seconde extrémité libre (152) du tube de courant descendant (146) et l'orifice d'entrée (134).

3. Appareil à circuit respiratoire (110) selon la revendication 2, en outre **caractérisé en ce que** l'élément déflecteur (154) et l'orifice d'entrée (134) sont espacés l'un de l'autre à une distance sélectionnée dans une plage comprise entre environ 0,019 millimètre et 0,036 millimètre.

4. Appareil à circuit respiratoire (110) selon la revendication 2, en outre **caractérisé en ce que** l'orifice d'entrée (134) définit un premier plan imaginaire et l'élément déflecteur (154) définit un second plan imaginaire, et les premier et second plans imaginaires sont opposés facialement et parallèles l'un à l'autre.

5. Appareil à circuit respiratoire (110) selon la revendication 2, en outre **caractérisé en ce qu'**une pluralité d'entretoises (156) relient la seconde extrémité libre (152) du tube de courant descendant (146) et l'élément déflecteur (154) et définissent des ouvertures d'entretoise (158) entre celles séquentielles des entretoises (156) de telle sorte que lorsque l'air ambiant pénètre dans le conteneur de circuit respiratoire (120) au travers de la valve d'entrée (122), l'air ambiant traverse le conduit de courant descendant (148) et en sort à travers les ouvertures d'entretoise (158).

6. Appareil à circuit respiratoire (110) selon la revendication 1, dans lequel l'appareil à circuit respiratoire (110) et le nébuliseur (112) sont en outre **caractérisés en ce qu'**un conteneur de nébuliseur (162), qui peut être connecté à une extrémité inférieure du conteneur de circuit respiratoire (120) et qui contient le dispositif du nébuliseur (112), assure le passage de l'aérosol entre le nébuliseur (112) et la chambre intérieure (128).

7. Appareil à circuit respiratoire (110) selon la revendication 6, dans lequel l'appareil à circuit respiratoire (110) et le nébuliseur (112) sont en outre **caractérisés en ce que** le conteneur de circuit respiratoire (120) et le conteneur du nébuliseur (160) peuvent être reliés de telle sorte que l'orifice d'entrée (134) du dispositif du nébuliseur (112) s'étend dans la chambre intérieure (128) pour assurer le passage de l'aérosol entre le dispositif du nébuliseur (112) et la chambre intérieure (128) de telle sorte qu'un premier plan imaginaire, défini par l'orifice d'entrée (134), et un second plan imaginaire, défini par l'élément déflecteur (154), sont opposés facialement et parallèles l'un à l'autre.

8. Appareil à circuit respiratoire (110) selon la revendication 7, dans lequel l'appareil à circuit respiratoire (110) et le nébuliseur (112) sont en outre **caractérisés en ce que** le dispositif du nébuliseur (112) comprend un premier élément conique orienté vers le haut avec un périmètre circonférentiel à une extrémité inférieure relié à une paroi intérieure du conteneur du nébuliseur (160) pour fournir un réservoir de liquide (130) entre une surface supérieure du premier élément conique et la paroi intérieure du conteneur du nébuliseur (160), et un passage de gaz central inférieur qui s'étend vers le haut à travers l'axe central vertical du premier élément conique, et
un second élément conique de forme creuse de telle sorte qu'une surface intérieure du second élément conique et la surface supérieure du premier élément conique forment un passage de liquide pour évacuer le liquide du réservoir de liquide et vers le haut entre le premier et le second éléments coniques vers l'extrémité supérieure du passage de gaz central inférieur dans le premier élément conique, le second élément conique comprenant un passage de gaz central supérieur qui s'étend vers le haut depuis le passage de gaz central inférieur et se terminant dans l'orifice d'entrée (134) et étant mécaniquement indépendant et extractible du conteneur du nébuliseur (160), du premier élément conique et de l'appareil à circuit respiratoire (110).
